# EUROPEAN PATENT SPECIFICATION

(11) **EP 4 044 888 B1**
(45) Date of publication and mention of the grant of the patent: **20.08.2025**
(21) Application number: 20718488.8
(22) Date of filing: 19.03.2020
(51) Int. Cl.: A47K 3/28, A61H 35/02

(54) **IMPROVEMENTS IN AND RELATING TO SAFETY SHOWERS AND EYE-WASHING APPARATUS**
VERBESSERUNGEN AN UND IN BEZUG AUF SICHERHEITSDUSCHEN UND AUGENWASCHVORRICHTUNGEN
PERFECTIONNEMENTS APPORTÉS ET SE RAPPORTANT À DES DOUCHES DE SÉCURITÉ ET APPAREIL DE RINÇAGE OCULAIRE

(30) Priority: 17.10.2019 GB 201915055
(43) Date of publication of application: 24.08.2022
(73) Proprietor: Hughes Safety Showers Limited, Bredbury, Cheshire SK6 2SS (GB); Nath, Keshar, Wilton, CT 06897 (US); Ludlaim, Alan, Bredbury Cheshire SK6 2SS (GB); Damji, Kiran, Bredbury Cheshire SK6 2SS (GB)
(72) Inventor: NATH, Keshar, Wilton, Connecticut 06897 (US); LUDLAIM, Alan, Bredbury Cheshire SK6 2SS (GB); DAMJI, Kiran, Bredbury Cheshire SK6 2SS (GB)
(74) Representative: Bardehle Pagenberg Partnerschaft mbB Patentanwälte Rechtsanwälte
(86) International application number: PCT/US2020/023736
(87) International publication number: WO 2021/076180

(56) References cited:
- GB-A- 1 300 125
- US-A1- 2013 021 167
- US-A1- 2017 228 999

## Description

### RELATED APPLICATIONS

This application claims the benefit of GB Patent Application No. 1915055.6, filed October 17, 2019.

### FIELD

The disclosure generally relates to safety shower stations and/or eye washing stations, such as emergency showers and/or eye washing stations, for containing and dispensing a shower, spray or jet of water with which to rinse, bathe or wash a part of a user's body and/or eyes. The invention is directed towards an emergency washing apparatus adapted for generating a controlled spray or jet of water directed at a user for washing therewith.

### BACKGROUND

In industrial settings, such as in chemical processing plants or the like, personnel may be exposed to potentially harmful chemicals or materials in liquid, gaseous, vapor or powder form from time to time. Should such harmful materials contaminate the skin and/or eyes of a member of personnel, then it is essential that the skin and/or eyes of the member of personnel are rinsed, bathed or washed as soon as possible and as a matter of urgency so as to remove the contaminant materials and thereby limit damage to them.

It is common practice that, in such industrial settings, emergency shower stations and/or eye washing stations are provided at which a supply of washing fluid (typically water) is stored for this use exclusively. Such emergency shower stations and/or eye washing stations may often consist of a water tank or reservoir to which is connected a water dispensing shower head or nozzle adapted for providing an output spray or jet of water to be directed over the user, or into the eyes of the user, for washing.

Given that long periods of time may pass between successive uses of these emergency shower/washing stations, un-used water is often found to accumulate and stagnate within the water tanks and/or water conduits of the apparatus between uses. This means that, any successive use of the shower/washing station will cause the eyes of the user to be based, at least initially, with stagnant water remaining from the previous use of the apparatus. This stagnant water is likely to contain harmful bacteria which could be very damaging to the health of the user. A further problem that can arise from long periods of inactivity is that the water flow control valves of a shower may seize up due to corrosion or an accumulation of
debris in between moving parts of the valve mechanism, over time, which impedes or prevents their proper movement.

In addition, in order to be effective it is necessary that a suitable rate of flow of water is output from the water outlet (e.g. shower head or eye-wash nozzle) sufficient to permit effective washing of the user's skin and/or eyes. Faults in the equipment could cause a user to suffer avoidable harm due to ineffective operation when needed most - e.g. in an emergency. Thus, it can be important that the equipment is regularly checked and tested in relevant ways to ensure that they remain compliant with local, national standards and safety regulations (e.g. European Standard "EN 15154 - Safety showers"; "ANSI Z358.1 Safety Shower & Eye Wash Regulations").

The present invention aims to address these matters.
US 2013/021167 A1 discloses a safety station for use in a process plant which includes one or more leverless limit switches to detect activation of one or more parts of the safety station. The safety station further includes a wireless transmitter coupled to the leverless limit switches to transmit signals associated with the safety station to a base station device, which is communicatively coupled to one or more control and/or monitoring devices.

### SUMMARY

The invention is defined by appended independent claim 1. The dependent claims define advantageous embodiments. The disclosure may provide an apparatus and method for remote monitoring and data capture in an emergency washing apparatus.

In a first aspect, the invention provides an emergency washing apparatus adapted for generating a controlled spray or jet of water directed at a user for washing therewith, the apparatus comprising: a washing unit including a water inlet and one or more water outlets arranged for forming the spray or jet of water received from the water inlet; one or more sensors configured to sense one or more properties of the water received at the water inlet selected from: a water flow rate; a water pressure; a water temperature; a water pH level, a bacteria content; wherein the one or more sensors are configured to generate a respective measurement signal indicating a value of the one or more sensed properties of the water, and to transmit the measurement signal for reception locally at, or remotely from, the washing unit.

The emergency washing system may include a shower control unit which is located locally at, or remote from, the washing unit and which is arranged to receive the respective measurement signals and to issue a warning signal if the received measurement signals fail to comply with respective pre-set conditions thereby indicating a malfunction of the washing unit.

The emergency washing system may include one or more remotely controllable water flow control valves each configured in fluid communication with the water inlet and with a respective one of the one or more water outlets, wherein each remotely controllable water flow control valve is operable to reversibly place a respective said water outlet in fluid communication with the water inlet. The shower control unit is preferably arranged to remotely control one or more of the remotely controllable water flow control valves to reversibly place a respective said water outlet in fluid communication with the water inlet.

The shower control unit may be arranged to issue a said warning signal if a plurality of the received measurement signals fail to comply concurrently with pre-set conditions whereby concurrent compliance with the pre-set conditions is such that a condition applied to one of the received measurement signals is conditional upon another said received monitoring signal.

The emergency washing system may include a shower control unit which is located locally at, or remote from, the washing unit and which is arranged to receive the respective measurement signals, wherein the shower control unit includes a data processor and a display unit configured to generate and display a graphical user interface displaying one or more of the sensed properties of the water.

The data processor and display unit are preferably configured to issue the warning signal as a warning displayed by the graphical user interface.

The washing unit may include a user identity unit configured to generate identification information identifying a user of the washing unit and to transmit the user identification information. Desirably, the emergency washing system includes a shower control unit located locally at, or remote from the washing unit and responsive to receipt of the transmitted user identification information to issue a telecommunications signal conveying information selected from: information identifying the location of the washing unit; and, information identifying the user.

The data processor and display unit may be configured to generate one or each of: geographical coordinates identifying the location of the washing unit; a map of an environment local to the washing unit upon which the location of the washing unit is shown, and to display the geographical coordinates or map on the graphical user interface. For example, the geographical coordinates may then be used to show the location of the washing unit on an online mapping application/software (e.g. Google Maps).

In a corresponding aspect, the disclosure may provide an emergency washing method for in an apparatus adapted for generating a controlled spray or jet of water directed at a user for washing therewith, the method comprising: providing a washing unit including a water inlet and one or more water outlets and therewith forming the spray or jet of water received from the water inlet; providing one or more sensors and therewith sensing one or more properties of the water received at the water inlet selected from: a water flow rate; a water pressure; a water temperature; a water pH level, a bacteria content; by the one or more sensors, generating a respective measurement signal indicating a value of the one or more sensed properties of the water, and transmitting the measurement signal for reception locally at, or remotely from, the washing unit.

The method may include providing a shower control unit which is located locally at, or remote from, the washing unit and therewith receiving the respective measurement signals and issuing a warning signal if the received measurement signals fail to comply with respective pre-set conditions thereby indicating a malfunction of the washing unit.

The method may include providing one or more remotely controllable water flow control valves each configured in fluid communication with the water inlet and with a respective one of the one or more water outlets, and remotely controlling one or more water flow control valves to reversibly place a respective water outlet in fluid communication with the water inlet. The shower control unit is preferably arranged to remotely control the one or more of the remotely controllable water flow control valves in this way.

The method may include, by the shower control unit, issuing a said warning signal if a plurality of the received measurement signals fail to comply concurrently with pre-set conditions whereby concurrent compliance with the pre-set conditions is such that a condition applied to one the received measurement signals is conditional upon another said received monitoring signal.

The method may include, providing a shower control unit which is located locally at, or remote, from the washing unit and therewith receiving the respective measurement signals and, by the shower control unit, generating and displaying a graphical user interface showing one or more of the sensed properties of the water.

The method may include issuing the warning signal as a warning displayed by the graphical user interface.

The method may include providing a user identity unit and therewith generating identification information identifying a user of the washing unit, and transmitting the user identification information, wherein the method includes providing a shower control unit located locally at, or remote from, the washing unit and therewith responding to receipt of the transmitted user identification information by issuing a telecommunications signal conveying: information identifying the location of the washing unit; and, information identifying the user.

The method may include, by the data processor, generating one or each of: geographical coordinates identifying the location of the washing unit; a map of an environment local to the washing unit upon which the location of the washing unit is shown, and displaying the geographical coordinates or map on the graphical user interface. For example, the geographical coordinates may then be used to show the location of the washing unit on an online mapping application/software (e.g. Google Maps).

The disclosure may provide an apparatus and method for remote activation of an emergency washing apparatus.

In a second aspect, the invention may provide an emergency washing apparatus adapted for generating a controlled spray or jet of water directed at a user for washing therewith, the apparatus comprising: a washing unit including a water inlet and one or more water outlets arranged for forming the spray or jet of water received from the water inlet; one or more remotely controllable water flow control valves each configured in fluid communication with the water inlet and with a respective one of the one or more water outlets, wherein each remotely controllable water flow control valve is operable to reversibly place a respective the water outlet in fluid communication with the water inlet; a shower control unit which is remote from the washing unit and which is arranged to remotely control the one or more remotely controllable water flow control valves to reversibly place a respective said water outlet in fluid communication with the water inlet.

The emergency washing apparatus may include a water flow-rate sensor arranged to measure a rate of flow of water from the water inlet to the one or more water outlets, and to generate a monitoring signal indicating a measured rate of flow. Desirably, the shower control unit is arranged to perform remote data gathering by: controlling at least one said remotely controllable water flow control valve to place a respective said water outlet in fluid communication with the water inlet for a finite pre-set time period; and, receiving the monitoring signal during the time period.

The shower control unit may be arranged to issue a warning signal if the measured rate of flow falls below a pre-set threshold water flow rate value.

The shower control unit may be arranged to repeat the remote data gathering if an interval of time since the most recent previous said remote data gathering exceeds a threshold time interval value.

In a corresponding aspect, the invention may provide an emergency washing method for an apparatus adapted for generating a controlled spray or jet of water directed at a user for washing therewith, the method comprising: providing a washing unit including a water inlet and one or more water outlets arranged for forming the spray or jet of water received from the water inlet; providing one or more remotely controllable water flow control valves each configured in fluid communication with the water inlet and with a respective one of the one or more water outlets; providing a shower control unit which is remote from the washing unit and therewith remotely controlling the one or more remotely controllable water flow control valves to reversibly place a respective said water outlet in fluid communication with the water inlet.

The method may include providing a water flow-rate sensor and therewith measuring a rate of flow of water from the water inlet to the one or more water outlets, and generating a monitoring signal indicating a measured rate of flow.

Desirably, the method includes, by the shower control unit, performing remote data gathering by: controlling at least one said remotely controllable water flow control valve to place a respective said water outlet in fluid communication with the water inlet for a finite pre-set time period; and, receiving the monitoring signal during the time period.

The method may include, by the shower control unit, issuing a warning signal if the measured rate of flow falls below a pre-set threshold water flow rate value.

The method may include, by the shower control unit, repeating the remote data gathering if an interval of time since the most recent previous said remote data gathering exceeds a threshold time interval value.

The disclosure may provide an apparatus and method for remote testing of an emergency washing apparatus according to multiple concurrent test criteria.

The disclosure may provide an apparatus and method for remote testing of an emergency washing apparatus according to multiple concurrent test criteria.

In a third aspect depending on the first aspect, the invention may provide an emergency washing apparatus adapted for generating a controlled spray or jet of water directed at a user for washing therewith, the apparatus comprising: a washing unit including a water inlet and one or more water outlets arranged for forming the spray or jet of water received from the water inlet; one or more water flow control valves each operably coupled to a respective control switch arranged to operate the water flow control valve to reversibly place a said water outlet in fluid communication with the water inlet; one or more sensors configured to detect an operation of a said control switch and to transmit a monitoring signal indicating an occurrence of such operation; one or more sensors configured to sense one or more properties of the water received at the water inlet selected from: a water flow rate; a water pressure; a water temperature; and to transmit a respective measurement signal indicating a sensed property of the water; a shower control unit which is located locally at, or remote from, the washing unit and which is arranged to receive the monitoring signals and the measurement signals and to issue a warning signal if the received monitoring signals and measurement signals collectively fail to comply concurrently with pre-set conditions thereby indicating a malfunction of the washing unit.

Preferably, the one or more said control switches are each selected from: manually operable control switches; remotely operable control switches.

Desirably, the concurrent compliance with the pre-set conditions is such that a condition applied to the received measurement signals is conditional upon the received monitoring signals.

The one or more sensors may be configured to sense the water flow rate and the shower control unit may be arranged to issue the warning signal if the measurement signal indicates a non-zero water flow rate and concurrently a said monitoring signal(s) indicates that no said water outlet is in fluid communication with the water inlet.

The one or more sensors may be configured to sense the water flow rate and the shower control unit may be arranged to issue the warning signal if the measurement signal indicates no water flow and concurrently a said monitoring signal(s) indicates that a said water outlet is in fluid communication with the water inlet.

The emergency washing apparatus may comprise a first said water outlet and a second said water outlet, in which the one or more sensors are configured to sense the water flow rate and the shower control unit is arranged to issue the warning signal if the measurement signal indicates a water flow rate exceeding: a first pre-set water flow rate threshold value and concurrently a said monitoring signal(s) indicates that only one of the first and second water outlets is in fluid communication with the water inlet; or, a second pre-set water flow rate threshold value exceeding the first pre-set water flow rate threshold value and concurrently a said monitoring signal(s) indicates that both of the first and second water outlets is in fluid communication with the water inlet.

The first pre-set water flow rate threshold value is preferably between 1 liter per minute and 12 liters per minute, or preferably between 1 liter per minute and 6 liters per minute, or preferably between 6 liters per minute and 12 liters per minute, or preferably be at least 6 liters per minute, or preferably at least 11 liters per minute (e.g. at least 11.4 liters per minute). The first pre-set water flow rate may be applicable to a desired water flow rate for an eye-wash apparatus, and one of the first and second water outlets may comprise an eye-wash nozzle(s). The second pre-set water flow rate threshold value is preferably between 30 liters per minute and 60 liters per minute, or preferably between 60 liters per minute and 100 liters per minute, or may be at least 100 liters per minute. The second pre-set water flow rate may be applicable to a desired water flow rate for a shower head, or for a shower head and an eye-wash apparatus when used concurrently, and one of the first and second water outlets may comprise a shower head, or the first and second water outlets may collectively comprise a shower head and an eye-wash apparatus having an eye-wash nozzle(s).

Accordingly, a lower flow rate is relevant to the use of the eyewash apparatus alone, and the first pre-set water flow rate threshold value may be associated with this. However, a higher flow rate is suitable for the use of the shower alone, while a yet higher flow rate is relevant to the concurrent use of both the eyewash and the shower together, and a respective second pre-set water flow rate threshold value may be selected to be applicable to each of these higher flow rates individually (e.g. a different second pre-set water flow rate threshold values each suitable to one of these two conditions respectively). One or each of the first pre-set water flow rate threshold value and the second pre-set water flow rate threshold value, may be compliant with any one of the following national standards: European Standard "EN 15154 - Safety showers"; "ANSI Z358.1 Safety Shower & Eye Wash Regulations").

The one or more sensors are preferably configured to sense the water pressure and the shower control unit is arranged to issue the warning signal if the measurement signal indicates a water pressure exceeding a pre-set upper pressure threshold value and concurrently a said monitoring signal(s) indicates that no said water outlet is in fluid communication with the water inlet. For example, this could indicate that the water control valve(s) are closed and that the water pressure 'behind' them is too high for safe use. For example, formation of a shower or eye-wash water spray of jet using a supply of water at such high pressure may be injurious to the user and/or may damage the equipment of the washing unit.

The one or more sensors are preferably configured to sense the water pressure and the shower control unit is arranged to issue the warning signal if the measurement signal indicates a water pressure less than a pre-set lower pressure threshold value, and concurrently a said monitoring signal(s) indicates that no said water outlet is in fluid communication with the water inlet. For example, this could indicate that the water control valve(s) are open and that the water pressure 'behind' them is too low for effective/safe use. For example, formation of a shower or eye-wash water spray of jet using a supply of water at such low pressure may be impossible or ineffective to produce the required spray /jet of water to allow the user to wash effectively.

The pre-set upper pressure threshold value may be between 200 KPa and 600 KPa (e.g. between 2 bar and 6 bar). The pre-set lower pressure threshold value may be between 200 KPa and 600 KPa (e.g. between 2 bar and 6 bar). For example, the pre-set upper pressure threshold value may be 600 KPa, whereas the pre-set lower pressure threshold value may be 200 KPa.

In a corresponding aspect, the disclosure may provide an emergency washing method adapted for generating a controlled spray or jet of water directed at a user for washing therewith, the method comprising: providing a washing unit including a water inlet and one or more water outlets arranged for forming the spray or jet of water received from the water inlet; providing one or more water flow control valves each operably coupled to a respective control switch arranged to operate the water flow control valve to reversibly place a said water outlet in fluid communication with the water inlet; detecting an operation of a said control switch and transmitting a monitoring signal indicating an occurrence of such operation; sensing one or more properties of the water received at the water inlet selected from: a water flow rate; a water pressure; a water temperature; and to transmit a respective measurement signal indicating a sensed property of the water; providing a shower control unit which is located locally at, or remote from, the washing unit and therewith receiving the monitoring signals and the measurement signals, and issuing a warning signal if the received monitoring signals and measurement signals collectively fail to comply concurrently with pre-set conditions thereby indicating a malfunction of the washing unit.

Preferably, the one or more control switches are each selected from: manually operable control switches; remotely operable control switches.

Desirably, the concurrent compliance with the pre-set conditions is such that a condition applied to the received measurement signals is conditional upon the received monitoring signals.

The method may include sensing the water flow rate issuing the warning signal if the measurement signal indicates a non-zero water flow rate and concurrently a said monitoring signal(s) indicates that no said water outlet is in fluid communication with the water inlet.

The method may include sensing the water flow rate and issuing the warning signal if the measurement signal indicates no water flow and concurrently a said monitoring signal(s) indicates that a said water outlet is in fluid communication with the water inlet.

The method may include providing a first said water outlet and a second said water outlet, and sensing the water flow rate and issuing the warning signal if the measurement signal indicates a water flow rate exceeding: a first pre-set water flow rate threshold value and concurrently a said monitoring signal(s) indicates that only one of the first and second water outlets is in fluid communication with the water inlet; or, a second pre-set water flow rate threshold value exceeding the first pre-set water flow rate threshold value and concurrently a said monitoring signal(s) indicates that both of the first and second water outlets is in fluid communication with the water inlet.

The first pre-set water flow rate threshold value is preferably between 1 liter per minute and 12 liters per minute, or between 6 liters per minute and 12 liters per minute. The second pre-set water flow rate threshold value is preferably be one of: between 60 liters per minute and 100 liters per minute; between 30 liters per minute and 60 liters per minute; at least 100 liters per minute.

The method may include sensing the water pressure and issuing the warning signal if the measurement signal indicates a water pressure exceeding a pre-set upper pressure threshold value and concurrently a said monitoring signal(s) indicates that no said water outlet is in fluid communication with the water inlet.

The method may include sensing the water pressure and issuing the warning signal if the measurement signal indicates a water pressure less than a pre-set lower pressure threshold value, and concurrently a said monitoring signal(s) indicates that no said water outlet is in fluid communication with the water inlet.

The disclosure may provide an apparatus and method for remote user ID capture and remote transmission to First Responders.

In a fourth aspect, the disclosure of may provide an emergency washing apparatus adapted for generating a controlled spray or jet of water directed at a user for washing therewith, the apparatus comprising: a washing unit including a water inlet and one or more water outlets arranged for forming the spray or jet of water received from the water inlet; a user identity apparatus configured to generate identification information identifying a user of the washing unit and to transmit the user identification information; a shower monitoring unit remote from the washing unit responsive to receipt of the transmitted user identification information to issue a telecommunications signal conveying: information identifying the location of the washing unit; and, information identifying the user.

Desirably, the user identity apparatus comprises any one or more of: a fingerprint detector configured for detecting a fingerprint of a user of the washing unit; a camera configured for detecting the face of a user of the washing unit; an RFID detector configured for detecting an RFID tag worn by a user of the washing unit. Facial recognition software may be used with the camera configured for detecting the face of a user of the washing unit, by applying the software to an image of the user as captured by the camera. Software may be used such as is readily apparent and available to the person of ordinary skill in the art.

Preferably, the washing unit includes a manually operable switch mechanism configured for activating the formation of the spray or jet of water, and the user identity apparatus includes the fingerprint detector arranged upon the manually operable switch and configured detect a fingerprint of a user in contact therewith.

Desirably, the shower monitoring unit includes a data processor and a display unit configured to generate one or each of: geographical coordinates identifying the location of the washing unit; a map of an environment local to the washing unit upon which the location of the washing unit is shown, and to display the geographical coordinates or map on the graphical user interface. For example, the geographical coordinates may then be used to show the location of the washing unit on an online mapping application/software (e.g. Google Maps).

Preferably, the shower monitoring unit is configured to generate one or each of: geographical coordinates identifying the location of the washing unit; a map of an environment local to the washing unit upon which the location of the washing unit is shown, and to convey the geographical coordinates or the map within the information identifying the location of the washing unit in the telecommunications signal. For example, the geographical coordinates may then be used to show the location of the washing unit on an online mapping application/software (e.g. Google Maps).

The shower monitoring unit may be configured to generate information selected from any one or more of: directions to the washing unit; medical data associated with the identity of the user, and to convey the information within the telecommunications signal.

In a corresponding aspect, the invention may provide an emergency washing method adapted for generating a controlled spray or jet of water directed at a user for washing therewith, the method comprising: providing a washing unit including a water inlet and one or more water outlets arranged for forming the spray or jet of water received from the water inlet; providing a user identity apparatus and therewith generating identification information identifying a user of the washing unit and transmitting the user identification information; providing a shower monitoring unit remote from the washing unit, and therewith receiving the transmitted user identification information and issuing a telecommunications signal conveying: information identifying the location of the washing unit; and, information identifying the user.

Desirably, the user identity apparatus comprises any one or more of: a fingerprint detector configured for detecting a fingerprint of a user of the washing unit; a camera configured for detecting the face of a user of the washing unit; an RFID detector configured for detecting an RFID tag worn by a user of the washing unit.

Preferably, the method includes providing a manually operable switch mechanism configured for activating the formation of the spray or jet of water, and providing a fingerprint detector upon the manually operable switch, and detecting a fingerprint of a user in contact therewith.

Desirably, the method includes providing a data processor and therewith generating one or each of: geographical coordinates identifying the location of the washing unit; a map of an environment local to the washing unit upon which the location of the washing unit is shown, and displaying the geographical coordinates or map on the graphical user interface. For example, the geographical coordinates may then be used to show the location of the washing unit on an online mapping application/software (e.g. Google Maps).

Preferably, the method includes generating one or each of: geographical coordinates identifying the location of the washing unit; a map of an environment local to the washing unit upon which the location of the washing unit is shown, and conveying the geographical coordinates or map within the information identifying the location of the washing unit in the telecommunications signal. For example, the geographical coordinates may then be used to show the location of the washing unit on an online mapping application/software (e.g. Google Maps).

The method may include generating information selected from any one or more of: directions to the washing unit; medical data associated with the identity of the user, and to convey the information within the telecommunications signal.

The invention may provide an apparatus and method for remote transmission of a geolocation of an emergency washing unit to First Responders.

In a fifth aspect, the invention may provide an emergency washing system adapted for generating a controlled spray or jet of water directed at a user for washing therewith, the apparatus comprising: a washing unit including a water inlet and one or more water outlets arranged for forming the spray or jet of water received from the water inlet; a positioning unit configured to generate positioning information identifying a location of the washing unit and to transmit the positioning information; a shower monitoring unit remote from the washing unit responsive to receipt of the transmitted positioning information to issue a telecommunications signal conveying information identifying the location of the washing unit.

The emergency washing apparatus may include a geo-spatial positioning transceiver unit operably connected to a Global Navigation Satellite System (GNSS) or a terrestrial wireless telecommunications network, and configured to determine the position of the washing unit accordingly.

The emergency washing apparatus may include user identity apparatus configured to generate identification information identifying a user of the washing unit and to transmit the user identification information; wherein the shower monitoring unit is responsive to receipt of the transmitted user identification information to issue a telecommunications signal conveying: information identifying the location of the washing unit; and, information identifying the user.

Desirably, the user identity apparatus comprises any one or more of: a fingerprint detector configured for detecting a fingerprint of a user of the washing unit; a camera configured for detecting the face of a user of the washing unit; an RFID detector configured for detecting an RFID tag worn by a user of the washing unit.

The washing unit may include a manually operable switch mechanism configured for activating the formation of the spray or jet of water, and the user identity apparatus may include the fingerprint detector arranged upon the manually operable switch and configured detect a fingerprint of a user in contact therewith.

Desirably, the shower monitoring unit includes a data processor and a display unit configured to generate one or both of: geographical coordinates, e.g. latitude and longitude, identifying the location of the washing unit; a map of an environment local to the washing unit upon which the location of the washing unit is shown, and to display the map, and/or the geographical coordinates, on the graphical user interface. For example, the geographical coordinates may then be used to show the location of the washing unit on an online mapping application/software (e.g. Google Maps).

Preferably, the shower monitoring unit is configured to generate one or both of: geographical coordinates, e.g. latitude and longitude, identifying the location of the washing unit; a map of an environment local to the washing unit upon which the location of the washing unit is shown, and to convey the geographical coordinates, and/or the map, within the information identifying the location of the washing unit in the telecommunications signal. For example, the geographical coordinates may then be used to show the location of the washing unit on an online mapping application/software (e.g. Google Maps).

The shower monitoring unit may be configured to generate information selected from any one or more of: directions to the washing unit; medical data associated with the identity of the user, and to convey the information within the telecommunications signal.

In a corresponding aspect, the invention may provide an emergency washing method adapted for generating a controlled spray or jet of water directed at a user for washing therewith, the method comprising: providing a washing unit including a water inlet and one or more water outlets arranged for forming the spray or jet of water received from the water inlet; providing a positioning unit and therewith generating positioning information identifying a location of the washing unit and transmitting the positioning information; providing a shower monitoring unit remote from the washing unit and therewith receiving the transmitted positioning information and issuing a telecommunications signal conveying information identifying the location of the washing unit.

The method may include providing a geo-spatial positioning transceiver unit operably connected to a Global Navigation Satellite System (GNSS) or a terrestrial wireless telecommunications network, and therewith determining the position of the washing unit accordingly. Examples of a Global Navigation Satellite System (GNSS) include the following: The United States' Global Positioning System (GPS); the Russian Federation's Global Orbiting Navigation Satellite System (GLONASS).

The method may include providing user identity apparatus and therewith generating identification information identifying a user of the washing unit, and transmitting the user identification information; and by the shower monitoring unit, responding to receipt of the transmitted user identification information by issuing a telecommunications signal conveying: information identifying the location of the washing unit; and, information identifying the user.

Desirably, the user identity apparatus comprises any one or more of: a fingerprint detector configured for detecting a fingerprint of a user of the washing unit; a camera configured for detecting the face of a user of the washing unit; an RFID detector configured for detecting an RFID tag worn by a user of the washing unit.

Preferably, the method includes providing a manually operable switch mechanism configured for activating the formation of the spray or jet of water, and providing a fingerprint detector upon the manually operable switch, and detecting a fingerprint of a user in contact therewith.

Desirably, the method includes providing a data processor and therewith generating one or both of: geographical coordinates, e.g. latitude and longitude, identifying the location of the washing unit; a map of an environment local to the washing unit upon which the location of the washing unit is shown, and displaying the map and/or the geographical coordinates on the graphical user interface. For example, the geographical coordinates may then be used to show the location of the washing unit on an online mapping application/software (e.g. Google Maps).

Preferably, the method includes generating one or both of: geographical coordinates, e.g. latitude and longitude, identifying the location of the washing unit; a map of an environment local to the washing unit upon which the location of the washing unit is shown, and conveying the map and/or the geographical coordinates within the information identifying the location of the washing unit in the telecommunications signal. For example, the geographical coordinates may then be used to show the location of the washing unit on an online mapping application/software (e.g. Google Maps).

The method may include generating information selected from any one or more of: directions to the washing unit; medical data associated with the identity of the user, and to convey the information within the telecommunications signal.

In another aspect, the invention provides an emergency washing system comprising a washing unit including a water tank for containing water, a water outlet comprising a shower head and/or a nozzle for outputting a spray or jet of water from the water tank, a water flow control part operable and arranged to reversibly place the water tank in fluid communication with the water outlet in response to either of: manual control of the washing unit, or a remote control signal. The washing unit also includes a sensor part configured to transmit monitoring signals each indicating a respective occurrence of said fluid communication. The emergency washing system further comprises a shower control unit which is remote from the washing unit and which is arranged to receive the monitoring signals and to issue the remote control signal to the water flow control part if an interval of time since receipt of the most recently received monitoring signal exceeds a threshold time interval value.

The emergency washing system preferably also includes a flow-rate sensor arranged to measure a rate of flow of water from the water tank to the water outlet. The monitoring signal preferably includes a measured rate of flow associated with a respective occurrence of the fluid communication. The shower control unit is preferably arranged to issue a warning signal if the measured rate of flow falls below a threshold water flow rate value.

The emergency washing system may include a wearable user identity tag containing user identification information configured for detection. The washing unit may comprise a detector responsive to proximity of the user identity tag to: detect the user identification information contained therein and, transmit the detected user identification information to the shower control unit. Desirably, the shower control unit is responsive to receipt of the transmitted user identification information to issue a telecommunications signal conveying information identifying the location of the washing unit, and information identifying the user.

In yet another aspect, the invention provides an emergency washing method in an emergency washing system comprising a washing unit including: a water tank for containing water, a water outlet comprising a shower head and/or a nozzle for outputting a spray or jet of water from the water tank, a water flow control part, and a sensor part; and a shower control unit remote from the washing unit. The method comprises the following steps performed by the components of the emergency washing system, as follows. By the water flow control part, reversibly placing the water tank in fluid communication with the water outlet in response to either of: manual control of the washing unit, or a remote control signal. By the sensor part, transmitting monitoring signals each indicating the sensing thereby of a respective occurrence of said fluid communication. By the shower control unit, receiving the monitoring signals and issuing the remote control signal to the water flow control part if an interval of time since receipt of the most recently received monitoring signal exceeds a threshold time interval value.

Desirably, in the emergency washing method the emergency washing system comprises a flow-rate sensor, and the method includes the following steps. By a flow-rate sensor, measuring a rate of flow of water from the water tank to the water outlet, wherein a said monitoring signal includes a said measured rate of flow associated with a respective occurrence of said fluid communication. By the shower control unit, issuing a warning signal if the measured rate of flow falls below a threshold water flow rate value.

The method may include providing a wearable user identity tag containing user identification information configured for detection and, at the washing unit, providing a detector responsive to proximity of the user identity tag and therewith detecting the user identification information contained therein. The method preferably includes transmitting the detected user identification information to the shower control unit and, by the shower control unit, in response to receipt of the transmitted user identification information, issuing a telecommunications signal conveying information identifying the location of the washing unit and information identifying the user.

The user identity apparatus, described above in any aspect, may comprise any one or more of: a fingerprint detector configured for detecting a fingerprint of a user of the washing unit; a camera configured for detecting the face of a user of the washing unit; an RFID detector configured for detecting an RFID tag worn by a user of the washing unit. Facial recognition software may be used with the camera configured for detecting the face of a user of the washing unit, by applying the software to an image of the user as captured by the camera. Software may be used such as is readily apparent and available to the person of ordinary skill in the art.

### BRIEF DESCRIPTION OF DRAWINGS

For a better understanding of the invention, and without intending to limit the scope of the invention, there now follows a description of some examples and embodiments of the invention with reference to the drawings, of which:
Fig.1 shows a schematic diagram illustrating an emergency washing system according to an embodiment of the invention;
Fig.2 shows a schematic diagram illustrating an emergency washing system according to an embodiment not forming part of the invention;
Fig.3 shows a user interface display of water flowrate, pressure and temperature data collected over an interval of time by the emergency washing system according to Fig.1 or Fig.2;
Fig.4A shows a user interface display of the status and spatial location of a plurality of the emergency washing systems each of which is according to Fig.1 or Fig.2;
Fig.4B shows a user interface display of the status and spatial location of a plurality of the emergency washing systems each of which is according to Fig.11or Fig.2;
Fig.5 shows a flow chart of steps in an emergency washing method in an emergency washing system according to Fig.1 or Fig.2;
Fig.6A shows a further flow chart of steps in an emergency washing method in an emergency washing system according to Fig.1 or Fig.2;
Fig.6B shows another flow chart of steps in an emergency washing method in an emergency washing system according to Fig.1 or Fig.2;
Fig.7 shows a flow chart of steps in an emergency washing method in an emergency washing system according to Fig.1 or Fig.2;

### DESCRIPTION OF EMBODIMENTS

In the drawings, like items are assigned like reference symbols.

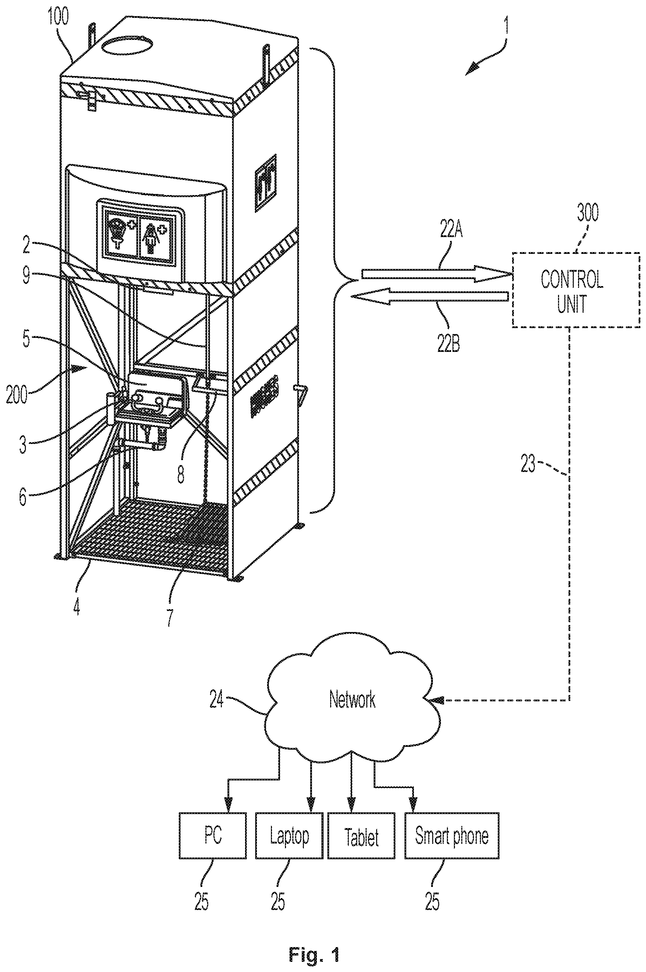

Referring to Fig.1, there is illustrated, in schematic form, an emergency washing system according to the invention. The emergency washing system includes a washing unit which comprises a water tank (100) and a washing cubicle (200) above which the water tank is disposed such that the floor of the water tank provides the ceiling of the washing cubicle. The water tank is configured to contain clean water for use in washing a user of the shower within the washing cubicle and, for this purpose, two separate water gravity-fed water outlets are disposed below the water tank within the washing cubicle, each of which is in fluid communication with the water tank.

A first of these two water outlets comprise a shower head (2) disposed in the ceiling of the shower cubicle which is configured to produce a downward shower spray pattern of water fed to it from the water tank, in use, directed towards the floor (4) of the washing cubicle. A second of the two water outlets is a nozzle unit (3) for outputting a pair of simultaneous upward sprays or jets of water from the water tank, at an eye-washing station located within the washing cubicle at about waist height (e.g. between 1.0m and 1.25m above ground level) and is connected in fluid communication with the water tank (100) via a water pipe line (6) which passes from the water tank to the nozzle unit (3). The nozzle unit is configured to produce two sprays/jets laterally separated by atypical human inter-ocular distance, and of substantially the same water output flow rate and pressure such that the water sprays/jets are of substantially the same shape, form and force. This permits both eyes of a user to be bathed in water simultaneously at the same rate of water flow to each eye.

The washing unit also includes water outlet control parts comprising a water outlet control valves (not shown) controllable to open and close to reversibly place the water tank in fluid communication with the shower head (2) and/or the eye-washing nozzle (3). The water outlet control parts are arranged to operate in this way in response to either of: a local, manual control operation of shower/nozzle on/off controls of the washing unit situated within the washing cubicle to manually switch on/off a flow of water; or a remote control signal issued remotely from the washing unit to the same effect.

In particular, the washing unit comprises a number of manual control parts (5, 7, 8, 9) contained within the washing cubicle and configured for manual operation by a user there. Each of the manual control parts is manually operable, in the manner of an on-off switch, to operate either a shower water valve switch (not shown) or an eye/face wash water valve switch (not shown). For example, the shower valve switch is mechanically connected to each of: a foot peddle (7); a hand lever (8); a pull-chain (9); such that manual operation of any of these devices will operate the shower water valve switch to reversibly place the shower head (2) in fluid communication with the water tank, or to terminate such fluid communication, depending upon whether or not the two are already in fluid communication when the switch is operated. Similarly, the eye/face wash valve is mechanically connected to a cover panel (5) manually pivotable to reveal the eye-washing nozzles such that manual operation of the cover panel will operate the eye/face wash water valve switch to reversibly place the eye-washing nozzle (3) in fluid communication with the water tank, or to terminate such fluid communication, depending upon whether or not the two are already in fluid communication when the switch is operated.

The washing unit also includes a plurality of sensor parts each configured to transmit monitoring signals which indicate a respective occurrence of the operation of an associated water valve switch for causing fluid flow from the water tank (100) to a respective one of the shower head (2) or the eye-wash nozzle unit (3). Each sensor is configured to detect when a valve switch is operated on/off via manual operation of a respective one of the manual control parts (5, 7, 8, 9). A sensor may be any suitable sensor for detecting the operation of a switch, such as may be readily available to the person skilled in the art. For example, each switch may comprise mechanical movement of a lever to open/close an associated valve, and the associated sensor may comprise a permanent magnet fixed to the lever so as to me moveable into/away-from proximity to a static magnet sensor responsive to detect the permanent magnet when in said proximity. Accordingly, detection or non-detection of the presence of the permanent magnet is indicative of whether the lever arm has been actuated to open or close the associated valve.

The washing unit also includes a flow-rate sensor assembly (not shown) arranged to measure a rate of flow of water from the water tank to the shower head (2) and to the eye wash nozzle unit (3).

The washing unit may comprise any one or more of the following sensors: (a) An ambient temperature sensor for measuring the ambient temperature around the washing unit; (b) A water level sensor for sensing the level of water in the water tank (100); (c) A water tank temperature sensor for measuring the temperature of water in the water tank; (d) An ambient light-level sensor for measuring the ambient light level at the washing unit; (e) A water inlet flow rate detector for measuring a rate of flow of water (e.g. refill water supply) into the water tank (100); (1) An inlet temperature sensor for measuring the temperature of water at the water inlet of the water tank; (g) A switch sensor for sensing operation of (or the status: 'on'/'off ) of a valve control switch coupled to the shower water valve; (h) A switch sensor for sensing operation of (or the status: 'on'/'off ) of a valve control switch coupled to the eye-wash water valve; (i) A flow-rate sensor arranged to measure a rate of flow of water from the water tank to the shower head (2) and/or to the eye-wash nozzle unit (3); and (j) A water pressure sensor arranged to measure the water pressure at the water outlet of the water tank.

The emergency washing system further comprises a shower control unit (300) which is located at a geographically remote location separated from the geographical location of the washing unit (100, 200). The shower control unit is arranged to receive the monitoring signals (22A) issued by the sensor part located within the washing unit (100, 200) and to generate and issue the remote control signal (22B) to the water flow control parts as appropriate.

For example, if more than a predetermined time has passed between uses of the washing apparatus (100, 200), then as a safety operation, the appropriate water flow control valves of the washing unit are remotely controlled to flush water from the water tank and through the shower head (2) and to the eye-wash nozzle unit (3). This not only serves to prevent stagnation of water in the system and the dangers that can bring, as discussed above, but also to allow measurement and/or monitoring signals to be remotely collected by the control unit (300) from any of the sensors arrayed within the washing unit (100).

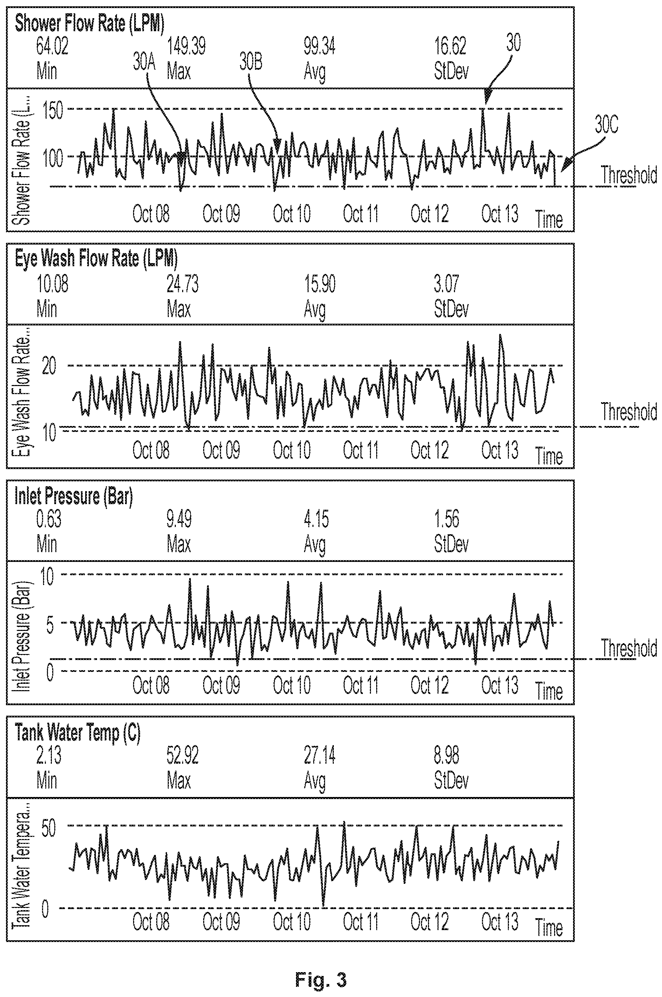

The measurement/monitoring signals (22A) include, for example, a measured rate of flow of water associated with the occurrence of fluid communication between the water tank (100) and one or both of the water outlets (2, 3). Fig.3 schematically shows an example of a time-sequence (30) of such measurement values and may represent the flow rate of water when e.g. the shower valve switch is open/on but the eye/face wash valve switch is closed/off, or vice versa, or e.g. when both the shower valve switch is open/on but the eye/face wash valve switch is open/on. This means that the measured flow rate may correspond to the rate of flow of water to the user e.g. via the shower head (2) alone, but not via the eye-wash nozzle unit (3), or vice versa. Of course, when the shower main valve switch is closed/off but the eye/face wash valve switch is open/on, then the measured flow rate may correspond to the rate of flow of water to the user via the eye-wash nozzle unit (3). The detected water flow rate is the flow rate detected over a continuous time interval (e.g. 1.5 minutes).

The shower control unit (300) is arranged to issue a warning signal (23) if the measured rate of flow falls below a threshold water flow rate value. As can be seen from Fig.3, over the time period in question, the measured flow rate, measured while the shower main valve switch is open/on, varies over the time interval and falls below the threshold value twice. The first occurrence (30A) is shortly after the beginning of the interval of time, just after the shower is switched on, and the second occurrence (30B) is approximately mid-way through the time interval. A third occurrence (30C) of below-threshold water flow rate occurs when the time interval ends as the shower is switched off by the user and the shower head (2) is no longer in fluid communication with the water tank (100). Of these three occurrences of below-threshold water flow rate, the first two occurrences (30A, 30B), cause the control unit to issue a warning signal (23). However, because the third occurrence of a below-threshold water flow rate does not happen when the shower head (2) is in fluid communication with the water tank (100), then the condition for issuing a warning signal is not met and no warning signal is issued. Thus, a condition required for issuance of a warning signal are that a below-threshold water flow rate measurement is received and that this flow rate measurement is concurrent with the water tank and water outlet (shower head or nozzle) being in fluid communication with each other.

The warning signals (23) are transmitted as telecommunications signals via a communications network (24), e.g. the Internet, to a systems operator, manager or other responsible personnel in charge of maintaining the emergency washing system. In particular, the warning signal is received at the communications device (25) used by the personnel in question. The communications device may be a desk-top computer, Tablet or Smartphone.

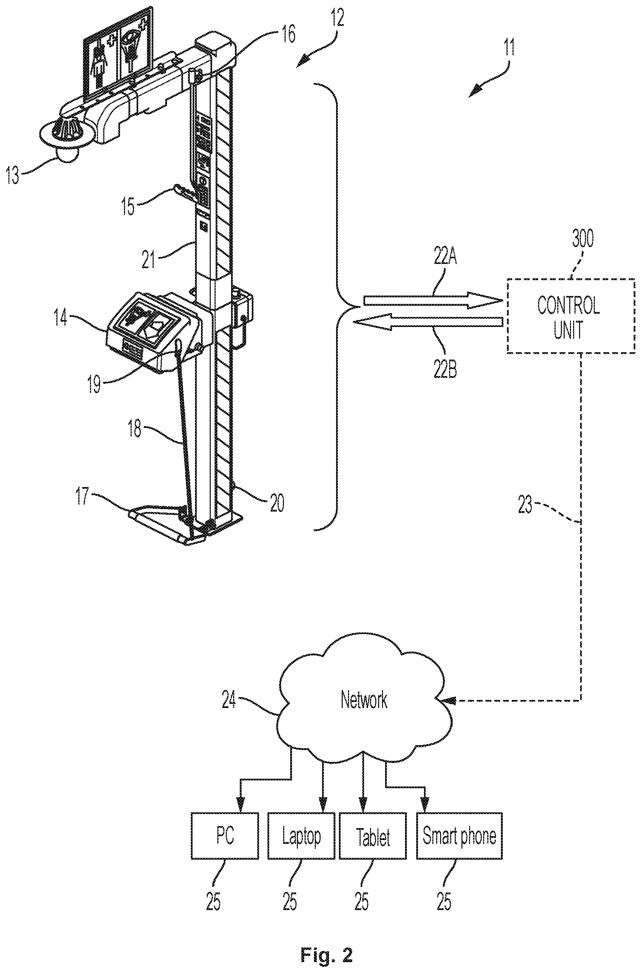

Referring to Fig.2, there is shown an emergency shower apparatus (11) which has the same function and features as the emergency shower apparatus described with reference to Fig.1, except for the provision of a mains water supply inlet at the base of the washing unit (12) in place of a water tank placed above the washing cubicle of the washing unit of Fig.1. In addition, the washing unit of Fig.2 comprises a stand-alone shower and eye-wash assembly with no washing cubicle. In particular, the washing unit (12) comprises a mains water inlet (20) located at the base of a column (21) housing water conduits leading from the mains water inlet to an eye-washing nozzle assembly housed underneath a flip-top cover (14), and to a shower head (13). The eye-washing nozzle assembly is served by a water flow control valve (not shown) housed within the washing unit in fluid communication with the mains water inlet (20) and is manually operable via a manual control switch mechanism (17, 18, 19) to open/close to place the eye-washing nozzle assembly in fluid communication with the mains water inlet (20). The manual control switch mechanism comprises a foot peddle (17) connected to a valve switch (19) via a pull-chain. Depression of the foot peddle by a user causes that pull-chain to pull down on the valve switch to operate it (e.g. open the valve). Similarly, the shower head (13) is served by a water flow control valve (not shown) housed within the washing unit in fluid communication with the mains water inlet (20) and is manually operable via a manual control switch mechanism (15) to open/close to place the shower head in fluid communication with the mains water inlet (20). The manual control switch mechanism comprises a pull-handle (15) connected to a valve switch (16) via a pull- rod. Downward pulling of the pull handle by a user causes that pull-rod to pull down on the valve switch (16) to operate it (e.g. open the valve).

All other functions, functionality and interactions (22A, 22B) of this washing unit (12) with the control unit (300) are as described herein with reference to Fig.1. In particular, the interaction (23) and functionality of the control unit (300) shown in Fig.2, with networks (24) and communications devices (25) are as described herein with reference to Fig.1.

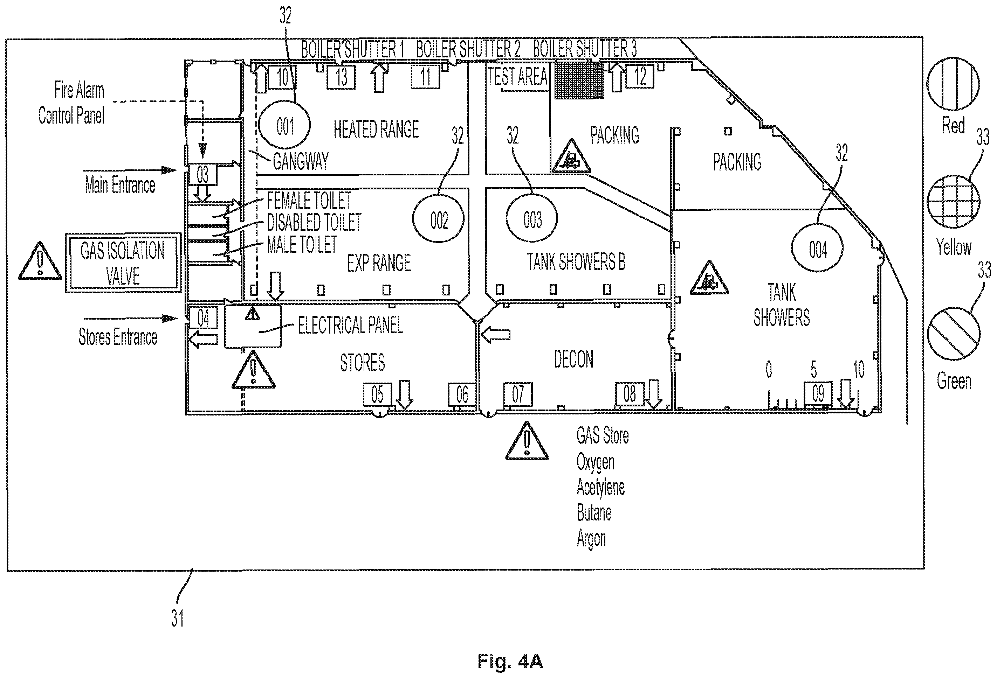

The communications device includes a display screen upon which the measurement data (30) may be displayed graphically as shown in Fig.4A. Furthermore, the communications device may be configured to display a schematic map of the geographical area within which the washing unit (100, 200) is situated, and in which the location and status of the washing unit is visually indicated. An example of this is illustrated in Fig.4A in which the geographical area is the floor of a chemical processing plant and the schematic map of that illustrates a map of the various chemical processing assemblies and units of the plant together with the separate locations of nine separate emergency washing units (unit numbers: 001, 002, ...009) each of which is according to the invention e.g. such as is shown in Fig.1.

A respective colored circle (32) surrounds the immediate location, upon the map, of a washing unit, together with the associated unit number. The color (33) of a given circle indicates whether a new warning signal has been received from the washing unit associated with that map location and unit number, and either has or has not yet been investigated/corrected. In the example given in Fig.4A, a red color indicates that a new warning signal has been received and remains un-investigated/corrected, while a green color indicates that no new warning signal has been received. A yellow color indicates that a new warning signal has been received and is currently under investigation for maintenance/ correction.

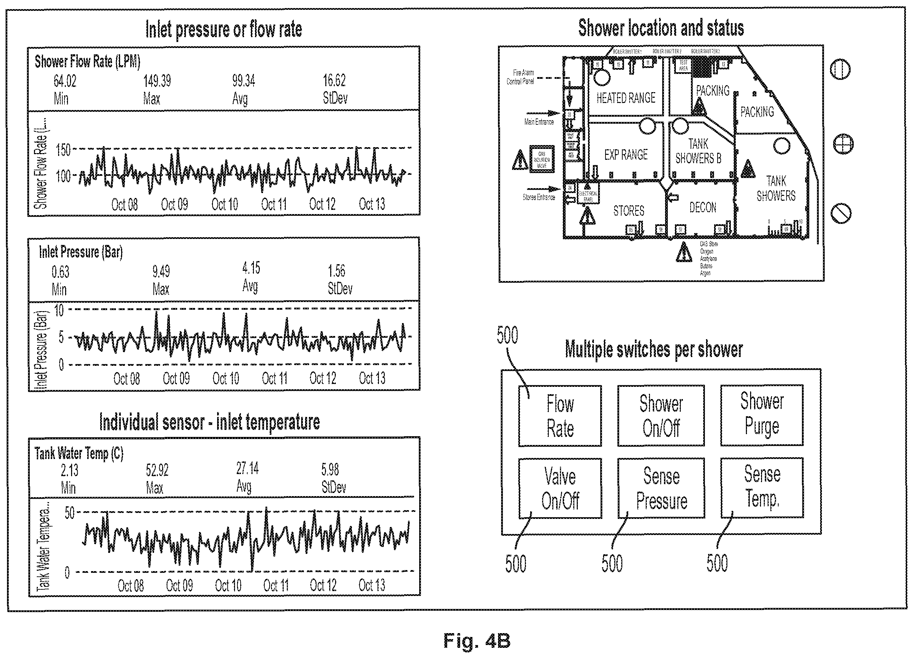

The emergency washing system may also include a wearable user identity tag (not shown) containing user identification information configured for detection. An example would be an RFID tag worn by an employee or personnel present on the plant floor and tasked with operating the plant machinery. The washing unit comprises an RFID detector unit (not shown) located within the washing cubicle (200; Fig.1), or the free-standing shower column (12, Fig.2) and is configured to be responsive to proximity of the user identity tag when the user is within the washing cubicle, or shower column. The RFID detector unit is configured to detect the user identification information contained within the RFID tag, and to transmit (22A) the detected user identification information to the shower control unit (300).
The shower control unit (300) is responsive to receipt of the transmitted user identification information to issue a telecommunications signal (23) conveying information identifying the washing unit in question (e.g. see Fig.4A, Fig.4B: unit #001, or #004 etc.) and the location of the washing unit (e.g. directions/coordinates according to the map of Fig.3), and information identifying the user.

The telecommunications signal is transmitted via a telecommunications network (24), such as the internet, to the communications device(s) of First-Responder (e.g. first aid) personnel associated with the chemical plant in which the washing unit (and the user of it) is located, and also (or alternatively) to the communications device(s) of ambulance/ emergency -services personnel. The information identifying the user may include relevant (possibly life-critical) medical information particular to the identified user, such as the equipment operated by the user within the plant (e.g. the particular dangerous chemicals he/she may have been exposed to), any medical allergies he/she may have, any existing medical conditions, his/her gender, age and other medical information which may assist first aid and/or ambulance personnel in more effectively helping the identified user of the emergency shower unit.

Fig.4B illustrates a graphical user interface (e.g. control 'dashboard') of a communications device of a systems operator, manager or other responsible personnel in charge of maintaining the emergency washing system. In particular, the monitoring/measurement signals (22A) are received at the communications device (25) used by the personnel in question. The communications device may be a desk-top computer, an iPad or an iPhone. The communications device comprises processor unit configured to receive the data conveyed by the monitoring/measurement signals and to generate a graphical user interface (GUI) configured to display the data graphically as shown in Fig.4B. For example, the received data may comprise time-series data relating to water pressure and/or water flow rate measurements, and water temperature. The GUI may also comprise one or more control switches presented as buttons (500) each responsive to a cursor selection^ click' upon the GUI to cause the control unit (300) to issue a specific control signal (22B) to the washing unit, such as to control the water flow control valves thereof.

In general, the embodiments of the invention illustrated in Fig.1 provides an emergency washing apparatus adapted for generating a controlled spray or jet of water directed at a user for washing with. The apparatus has a washing unit which may be as shown in Gig.1 or as shown in Fig.2, or another arrangement, each including at least a water inlet for receiving water (e.g. from a tank as in Fig.1, or from a mains water supply as in Fig.2) and one or more water outlets (e.g. a shower head and an eye-wash nozzle apparatus) arranged for forming the spray or jet of water received from the water inlet.
In preferred embodiments, such as those described above with reference to Fig.1 or Fig.2, the apparatus includes one or more sensors configured to sense one or more properties of the water received at the water inlet. These properties may be any one or more selected from: a water flow rate (using a flow rate sensor); a water pressure (using a pressure sensor); a water temperature (using a temperature sensor); a water pH level (using a pH sensor), a bacteria content (using a bacteria detector). The detectors used for this purpose may be any suitable detector such as would be readily apparent and available to the person of ordinary skill in the art.

In preferred embodiments, such as those described above with reference to Fig.1 or Fig.2, the one or more sensors are configured to generate a respective measurement signal indicating a value of the one or more sensed properties of the water, and to transmit the measurement signal for reception remotely from the washing unit. The transmitted measurement signal may be transmitted electronically, optically (e.g. fiber-optically) or wirelessly (e.g. a radio/telecoms signal). The transmission may be received by the control unit described above for use in monitoring and/or implementing control operations upon the washing unit. For example, the shower control unit may be arranged to issue a warning signal if the received measurement signals fail to comply with respective pre-set conditions thereby indicating a malfunction of the washing unit. The shower control unit may be arranged to generate a graphical user interface (GUI) arranged to display the measurements graphically on a display unit (e.g. a PC, or laptop etc.).

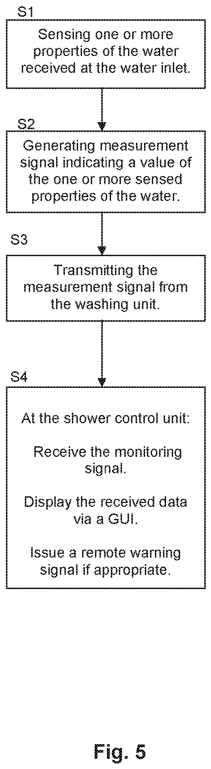

For example, Fig.5 illustrates the steps in an emergency washing method implemented by the emergency washing system described above. The method comprises the following steps performed by the components of the emergency washing system, as follows.

In a first step SI: Sensing one or more properties of the water received at the water inlet.

In a second step S2: Generating measurement signal indicating a value of the one or more sensed properties of the water.

In a third step S2: Transmitting the measurement signal from the washing unit.

In a fourth step S4: By the shower control unit: receiving the monitoring signal and/or displaying the received data via a GUI and/or issuing a remote warning signal if appropriate.

In preferred embodiments, one or more remotely controllable water flow control valves of the washing unit (Fig.1 or Fig.2) are each remotely controlled to reversibly place a respective the water outlet (e.g. a shower head, an eye-wash nozzle) in fluid communication with the water inlet (e.g. water tank outlet, mains water supply input port). In these embodiments, the shower control unit may be arranged to remotely detect when the water flow control valves are locally, manually operated (opened) by a user of the washing unit and therefore remote operation of the valves is not necessary. Alternatively, the shower control unit may be arranged to remotely control the one or more water flow control valves to reversibly place a respective water outlet in fluid communication with the water inlet. In some embodiments, a water flow-rate sensor is arranged to measure a rate of flow of water from the water inlet to the one or more water outlets, and to generate a monitoring signal indicating a measured rate of flow. The shower control unit is then arranged to perform remote data gathering by controlling the remotely controllable water flow control valves to place a respective water outlet in fluid communication with the water inlet for a finite pre-set time period. The shower control unit is arranged to receive the monitoring signals during the time period.

The shower control unit may issue a warning signal if the measured rate of flow falls below a pre-set threshold water flow rate value, and may repeat the remote data gathering if an interval of time since the most recent previous said remote data gathering exceeds a threshold time interval value.

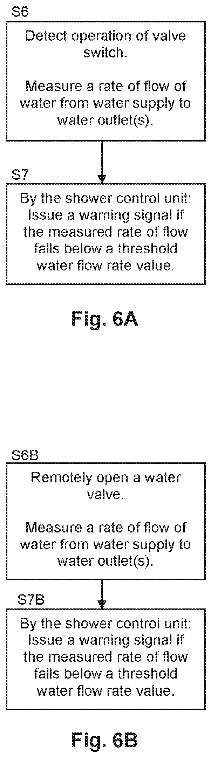

Fig.6A illustrates the steps in an emergency washing method implemented by the emergency washing system described above. The method comprises the following steps performed by the components of the emergency washing system, as follows.

In a first step S6: By the emergency shower unit, detect operation of a water flow control valve switch, and in response, measure a rate of flow of water from the water supply (e.g. water tank) to the water outlet (shower head or eye-wash nozzle).

In a second step S7: By the shower control unit, issue a warning signal if the measured rate of flow falls below a threshold water flow rate value.

This methodology applies when the water flow control valves are locally, manually operated (opened) by a user of the washing unit and therefore remote operation of the valves is not necessary.

Fig.6B illustrates the steps in an emergency washing method implemented by the emergency washing system described above. The method comprises the following steps performed by the components of the emergency washing system, as follows.

In a first step S6B: By the emergency shower unit, remotely operate a water flow control valve switch to 'open' it, and subsequently, measure a rate of flow of water from the water supply (e.g. water tank) to the water outlet (shower head or eye-wash nozzle).

In a second step S7B: By the shower control unit, issue a warning signal if the measured rate of flow falls below a threshold water flow rate value.

This methodology applies when the water flow control valves are not locally, manually operated (opened) by a user of the washing unit and therefore remote operation of the valves is necessary.

The methodology, according to Fig.6A or Fig.6B, may include sensing one or more properties of the water such as a water flow rate, and/or a water pressure, and the shower control unit issue a warning signal if the received measurement signals fail to comply with pre-set conditions thereby indicating a malfunction of the washing unit. For example, the pre-set conditions may require that if all water flow control valves are 'closed', then the water flow measurement indicates no water flow. For example, the pre-set conditions may require that if one, but not all, water flow control valves are 'open', then the measured flow rate should be above a pre-set flow rate and/or a measured water pressure is within a pre-set pressure range.

The pre-set rates/ranges may be specific to which of the water flow control valves are open and which are closed. This mirrors that requirement that required water flow rates and pressures differ depending on which one of the shower and the eye-wash apparatus is in use, or if both are in use.

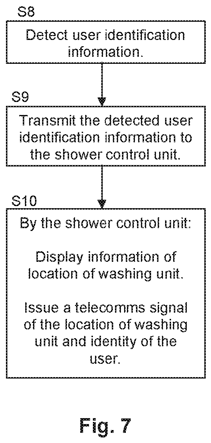

Fig.7 illustrates the steps in an emergency washing method implemented by the emergency washing system described above. The method comprises the following steps performed by the components of the emergency washing system, as follows.

In a first step S8: By the emergency shower unit, detect user identification information.

In a second step S9: By the emergency shower unit, transmit the detected user identification information to the shower control unit.

In a third step S10: By the shower control unit, issuing a telecommunications signal conveying the location of the washing unit and identity of the user.

It will be appreciated that the examples of embodiments of the invention, described above, include examples of decision logic applied by the control unit to remote measurements taken from the washing unit (Fig.1, Fig.2). These examples are not intended to be exhaustive, and other decision logic operations may be applied by the control unit. Table 1, shown below, illustrates a more detailed set of possible measurements gathered by the control unit, according to preferred embodiments, and examples of the applied decision logic that may be applied to the measurements by the control unit, in the form: 'If condition X occurs, then implement response T . Abbreviation 'PIR' refer to 'Passive Infra-Red'.

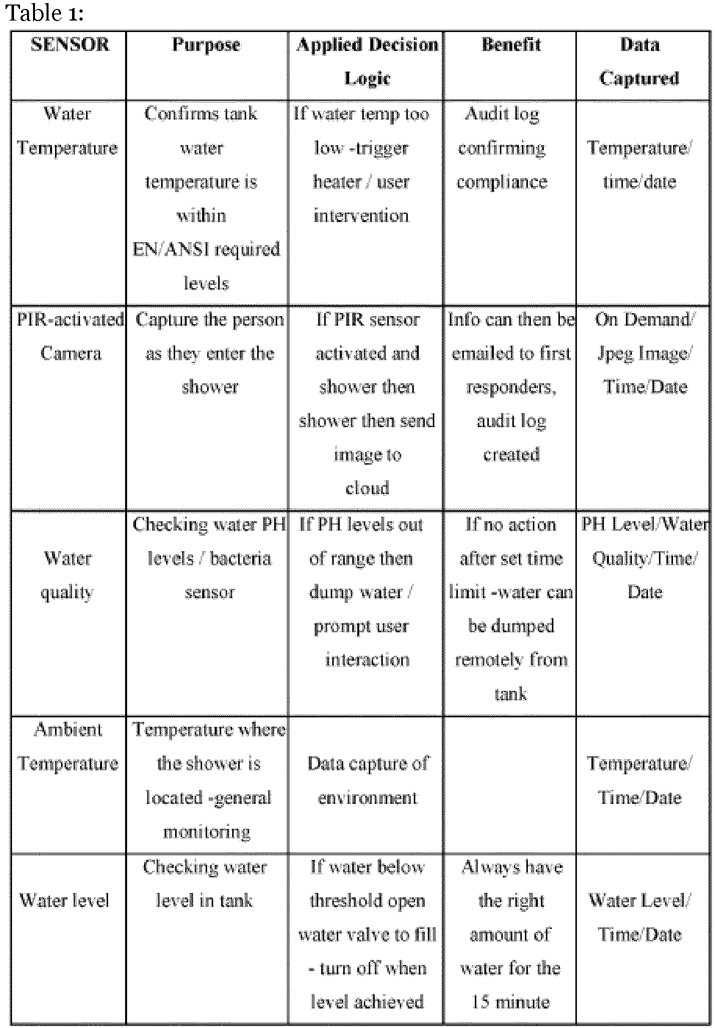

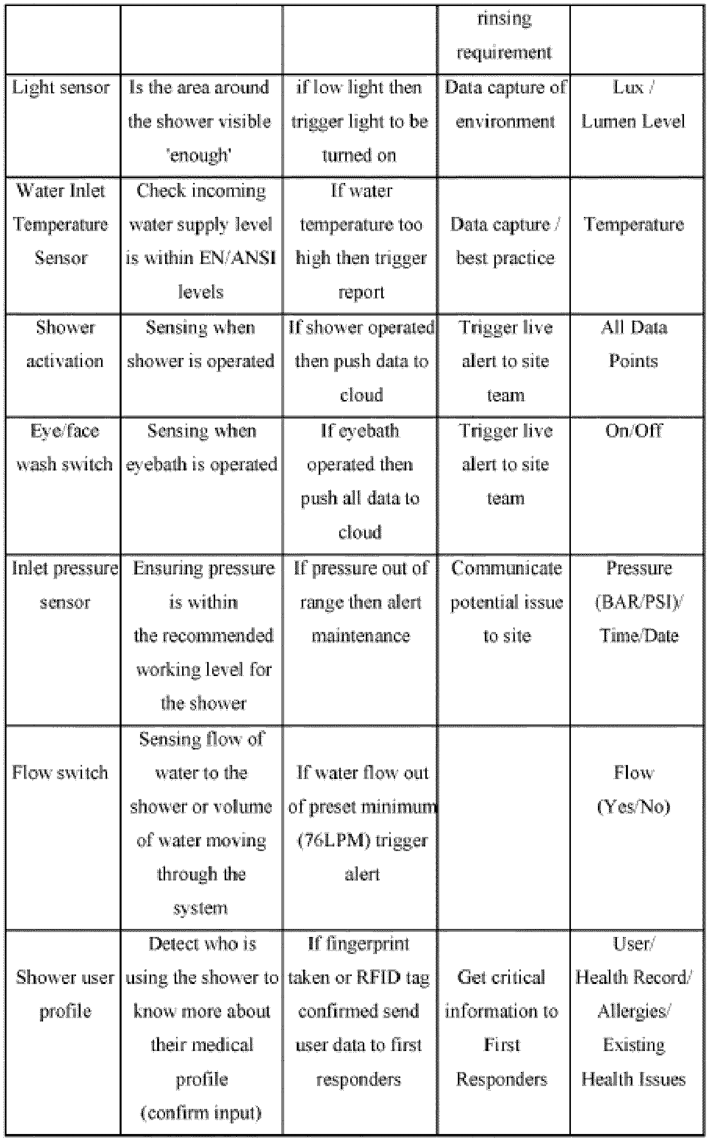

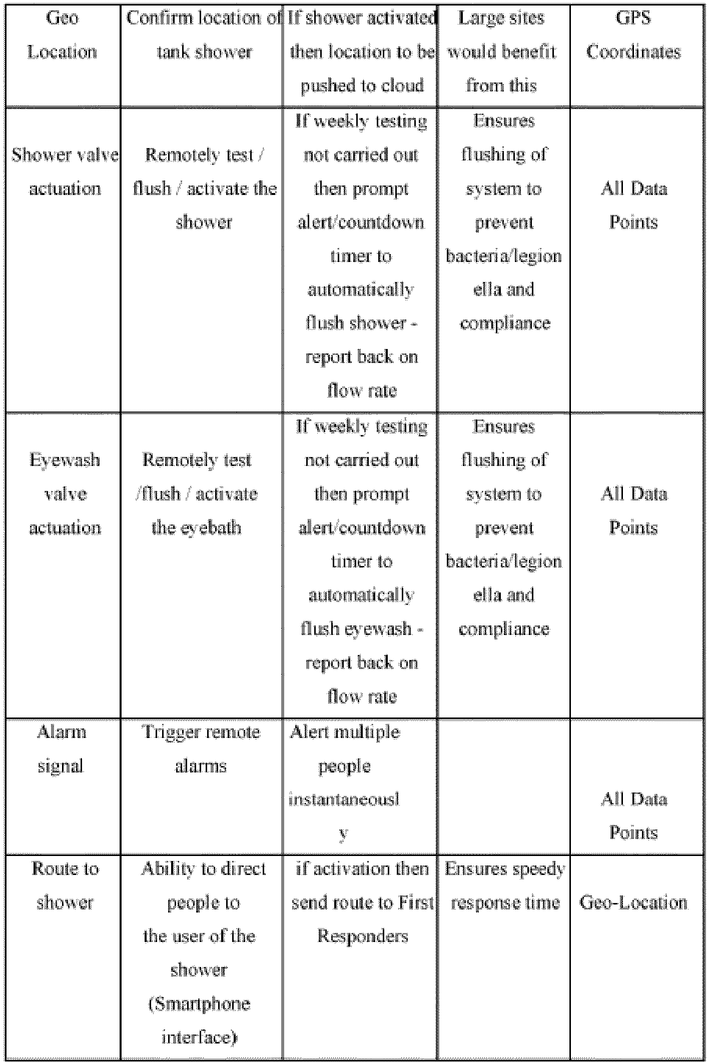

## Claims

1. An emergency washing apparatus adapted for generating a controlled spray or jet of water directed at a user for washing therewith, the emergency washing apparatus comprising:
a washing unit including a water inlet (20) and one or more water outlets (2) arranged for forming said spray or jet of water received from the water inlet (20);
the washing unit comprising further a water tank (100) and a washing cubicle (200) above which the water tank is disposed, and the water tank being fluidly connected to the water inlet (20);
one or more sensors positioned at the water inlet of the water tank (100) and configured to sense one or more properties of the water received at the water inlet (20) from the water tank (100), the properties selected from: a water flow rate, a water pressure, a water temperature, a water pH level, a bacteria content;
wherein the one or more sensors are configured to generate a respective measurement signal (22A) indicating a value of the one or more sensed properties of the water, and to transmit the measurement signal (22A) for reception locally at, or remotely from, the washing unit.

2. An emergency washing apparatus according to claim 1, including a shower control unit (300) which is located locally at, or remote from, the washing unit and which is arranged to receive said respective measurement signals (22A) and to issue a warning signal (23) if said received measurement signals (22A) fail to comply with respective pre-set conditions thereby indicating a malfunction of the washing unit.

3. An emergency washing apparatus according to claim 2, including:
one or more remotely controllable water flow control valves each configured in fluid communication with the water inlet (20) and with a respective one of said one or more water outlets, wherein each remotely controllable water flow control valve is operable to reversibly place a respective said water outlet in fluid communication with the water inlet (20);
wherein the shower control unit (300) is arranged to remotely control said one or more remotely controllable water flow control valves to reversibly place a respective said water outlet in fluid communication with the water inlet (20).

4. An emergency washing apparatus according to claim 2 or claim 3, in which the shower control unit (300) is arranged to issue a said warning signal (23) if a plurality of said received measurement signals (22A) fail to comply concurrently with pre-set conditions whereby concurrent compliance with said pre-set conditions is such that a condition applied to one said received measurement signals (22A) is conditional upon another said received monitoring signal (22A).

5. An emergency washing apparatus according to any of claims 2 to 4, wherein the shower control unit (300) is located locally at, or remote from, the washing unit and which is arranged to receive said respective measurement signals (22A), wherein the shower control unit (300) includes a data processor and a display unit configured to generate and display a graphical user interface displaying one or more of said sensed properties of the water.

6. An emergency washing apparatus according to claim 5 or any preceding claims, wherein the data processor and the display unit are configured to issue said warning signal (23) as a warning displayed by said graphical user interface.

7. An emergency washing apparatus according to claim 3, wherein the one or more water flow control valves are each operably coupled to a respective control switch arranged to operate the one or more water flow control valves to reversibly place the water outlet in fluid communication with the water inlet (20);
one or more sensors configured to detect an operation of a said control switch and to transmit a monitoring signal (22A) indicating an occurrence (30A-C) of the operation; and
a shower control unit (300) which is located locally at, or remote from, the washing unit and which is arranged to receive said monitoring signals (22A) and said measurement signals (22A) and to issue a warning signal (23) if said received monitoring signals (22A) and measurement signals (22A) collectively fail to comply concurrently with pre-set conditions thereby indicating a malfunction of the washing unit.

8. An emergency washing apparatus according to claim 7, in which the one or more said control switches are each selected from: manually operable control switches.

9. An emergency washing apparatus according to claim 1, including a water flow-rate sensor arranged to measure a rate of flow of water from said water inlet (20) to said one or more water outlets, and to generate a monitoring signal (22A) indicating a measured rate of flow;
wherein the shower control unit (300) is arranged to perform remote data gathering by:
controlling at least one said remotely controllable water flow control valve to place a respective said water outlet in fluid communication with the water inlet (20) for a finite pre-set time period; and,
receiving said monitoring signal (22A) during said time period.

10. An emergency washing apparatus according to claim 9, wherein the shower control unit (300) is arranged to repeat said remote data gathering if an interval of time since the most recent previous said remote data gathering exceeds a threshold time interval value.

11. An emergency washing apparatus according to claim 7, comprising a first said water outlet and a second said water outlet, in which the one or more sensors are configured to sense said water flow rate and the shower control unit (300) is arranged to issue said warning signal (23) if said measurement signal (22A) indicates said water flow rate exceeding:
a first pre-set water flow rate threshold value and concurrently a said monitoring signal(s) (22A) indicates that only one of the first and second water outlets is in fluid communication with said water inlet (20); or,
a second pre-set water flow rate threshold value exceeding the first pre-set water flow rate threshold value and concurrently a said monitoring signal(s) (22A) indicates that both of the first and second water outlets is in fluid communication with said water inlet (20).

12. An emergency washing apparatus according to claim 11, in which the first pre-set water flow rate threshold value is between 6 liters per minute and 12 liters per minute, and the second pre-set water flow rate threshold value is one of:
between 60 liters per minute and 100 liters per minute; between 30 liters per minute and 60 liters per minute; at least 100 liters per minute.

13. An emergency washing apparatus according to any preceding claim, in which the washing unit includes a user identity unit configured to generate identification information identifying a user of the washing unit and to transmit the user identification information;
wherein the emergency washing apparatus includes a shower control unit (300) located locally at, or remote from, the washing unit and responsive to receipt of the transmitted user identification information to issue a telecommunications signal conveying:
information identifying a location of the washing unit; and, information identifying the user.

14. An emergency washing apparatus according to claim 13, wherein the user identity apparatus comprises any one or more of: a fingerprint detector configured for detecting a fingerprint of the user of the washing unit; a camera configured for detecting the face of the user of the washing unit; an RFID detector configured for detecting an RFID tag worn by the user of the washing unit.

15. An emergency washing apparatus according to claim 13, wherein the data processor and the display unit are configured to generate one or each of: geographical coordinates identifying the location of the washing unit; a map of an environment local to the washing unit upon which the location of the washing unit is shown, and to display the geographical coordinates or the map on said graphical user interface.

## Patentansprüche

1. Notwaschvorrichtung, die angepasst ist, um einen gesteuerten Sprühstrahl oder Wasserstrahl zu erzeugen, der auf einen Benutzer gerichtet ist, um sich damit zu waschen, wobei die Notwaschvorrichtung Folgendes umfasst:
eine Wascheinheit, die einen Wassereinlass (20) und einen oder mehrere Wasserauslässe (2) beinhaltet, die angeordnet sind, um den Sprühstrahl oder Wasserstrahl zu bilden, der von dem Wassereinlass (20) empfangen wird;
wobei die Wascheinheit ferner einen Wassertank (100) und eine Waschkabine (200) umfasst, über der der Wassertank angeordnet ist, und wobei der Wassertank fluidisch mit dem Wassereinlass (20) verbunden ist;
einen oder mehrere Sensoren, die an dem Wassereinlass des Wassertanks (100) positioniert sind und konfiguriert sind, um eine oder mehrere Eigenschaften des Wassers zu erfassen, das an dem Wassereinlass (20) von dem Wassertank (100) empfangen wird, wobei die Eigenschaften ausgewählt sind aus: einer Wasserdurchflussrate, einem Wasserdruck, einer Wassertemperatur, einem WasserpH-Wert, einem Bakteriengehalt;
wobei der eine oder die mehreren Sensoren konfiguriert sind, um ein jeweiliges Messsignal (22A) zu erzeugen, das einen Wert der einen oder der mehreren erfassten Eigenschaften des Wassers anzeigt, und um das Messsignal (22A) zum Empfang lokal an oder entfernt von der Wascheinheit zu übertragen.

2. Notwaschvorrichtung nach Anspruch 1, die eine Duschsteuereinheit (300) beinhaltet, die sich lokal an oder entfernt von der Wascheinheit befindet und die angeordnet ist, um die jeweiligen Messsignale (22A) zu empfangen und ein Warnsignal (23) auszugeben, wenn die empfangenen Messsignale (22A) jeweilige voreingestellte Bedingungen nicht erfüllen, wodurch eine Fehlfunktion der Wascheinheit angezeigt wird.

3. Notwaschvorrichtung nach Anspruch 2, die Folgendes beinhaltet:
ein oder mehrere fernsteuerbare Wasserdurchflusssteuerventile, die jeweils in Fluidverbindung mit dem Wassereinlass (20) und mit einem jeweiligen des einen oder der mehreren Wasserauslässe konfiguriert sind, wobei jedes fernsteuerbare Wasserdurchflusssteuerventil betreibbar ist, um einen jeweiligen Wasserauslass reversibel in Fluidverbindung mit dem Wassereinlass (20) zu bringen;
wobei die Duschsteuereinheit (300) angeordnet ist, um das eine oder die mehreren fernsteuerbaren Wasserdurchflusssteuerventile fernzusteuern, um einen jeweiligen Wasserauslass reversibel in Fluidverbindung mit dem Wassereinlass (20) zu bringen.

4. Notwaschvorrichtung nach Anspruch 2 oder Anspruch 3, wobei die Duschsteuereinheit (300) angeordnet ist, um ein Warnsignal (23) auszugeben, wenn eine Vielzahl der empfangenen Messsignale (22A) gleichzeitig voreingestellte Bedingungen nicht erfüllen, wodurch die gleichzeitige Einhaltung der voreingestellten Bedingungen derart ist, dass eine Bedingung, die auf eines der empfangenen Messsignale (22A) angewendet wird, von einem anderen empfangenen Überwachungssignal (22A) abhängig ist.

5. Notwaschvorrichtung nach einem der Ansprüche 2 bis 4, wobei sich die Duschsteuereinheit (300) lokal an oder entfernt von der Wascheinheit befindet und die angeordnet ist, um die jeweiligen Messsignale (22A) zu empfangen, wobei die Duschsteuereinheit (300) einen Datenprozessor und eine Anzeigeeinheit beinhaltet, die konfiguriert sind, um eine grafische Benutzeroberfläche zu erzeugen und anzuzeigen, die eine oder mehrere der erfassten Eigenschaften des Wassers anzeigt.

6. Notwaschvorrichtung nach Anspruch 5 oder einem der vorhergehenden Ansprüche, wobei der Datenprozessor und die Anzeigeeinheit konfiguriert sind, um das Warnsignal (23) als eine Warnung auszugeben, die durch die grafische Benutzeroberfläche angezeigt wird.

7. Notwaschvorrichtung nach Anspruch 3, wobei das eine oder die mehreren Wasserdurchflusssteuerventile jeweils betriebsfähig mit einem jeweiligen Steuerschalter gekoppelt sind, der angeordnet ist, um das eine oder die mehreren Wasserdurchflusssteuerventile zu betreiben, um den Wasserauslass reversibel in Fluidverbindung mit dem Wassereinlass (20) zu bringen;
einen oder mehrere Sensoren, die konfiguriert sind, um eine Betätigung des Steuerschalters zu erfassen und um ein Überwachungssignal (22A) zu übertragen, das ein Auftreten (30A-C) der Betätigung anzeigt; und
eine Duschsteuereinheit (300), die sich lokal an oder entfernt von der Wascheinheit befindet und die angeordnet ist, um die Überwachungssignale (22A) und die Messsignale (22A) zu empfangen und ein Warnsignal (23) auszugeben, wenn die empfangenen Überwachungssignale (22A) und Messsignale (22A) gemeinsam gleichzeitig voreingestellte Bedingungen nicht erfüllen, wodurch eine Fehlfunktion der Wascheinheit angezeigt wird.

8. Notwaschvorrichtung nach Anspruch 7, wobei der eine oder die mehreren Steuerschalter jeweils ausgewählt sind aus: manuell betätigbaren Steuerschaltern.

9. Notwaschvorrichtung nach Anspruch 1, die einen Wasserdurchflussratensensor beinhaltet, der angeordnet ist, um eine Durchflussrate von Wasser von dem Wassereinlass (20) zu dem einen oder den mehreren Wasserauslässen zu messen und um ein Überwachungssignal (22A) zu erzeugen, das eine gemessene Durchflussrate anzeigt;
wobei die Duschsteuereinheit (300) angeordnet ist, um Ferndatenerfassung durchzuführen durch:
Steuern mindestens eines fernsteuerbaren Wasserdurchflusssteuerventils, um einen jeweiligen Wasserauslass für eine endliche voreingestellte Zeitdauer in Fluidverbindung mit dem Wassereinlass (20) zu bringen; und
Empfangen des Überwachungssignals (22A) während der Zeitdauer.

10. Notwaschvorrichtung nach Anspruch 9, wobei die Duschsteuereinheit (300) angeordnet ist, um die Ferndatenerfassung zu wiederholen, wenn ein Zeitintervall seit der letzten vorhergehenden Ferndatenerfassung einen Schwellenzeitintervallwert überschreitet.

11. Notwaschvorrichtung nach Anspruch 7, die einen ersten Wasserauslass und einen zweiten Wasserauslass umfasst, wobei der eine oder die mehreren Sensoren konfiguriert sind, um die Wasserdurchflussrate zu erfassen, und die Duschsteuereinheit (300) angeordnet ist, um das Warnsignal (23) auszugeben, wenn das Messsignal (22A) anzeigt, dass die Wasserdurchflussrate Folgendes überschreitet:
einen ersten voreingestellten Wasserdurchflussratenschwellenwert und gleichzeitig ein oder mehrere Überwachungssignale (22A) anzeigen, dass nur einer des ersten und des zweiten Wasserauslasses in Fluidverbindung mit dem Wassereinlass (20) steht; oder
einen zweiten voreingestellten Wasserdurchflussratenschwellenwert, der den ersten voreingestellten Wasserdurchflussratenschwellenwert überschreitet, und gleichzeitig ein oder mehrere Überwachungssignale (22A) anzeigen, dass sowohl der erste als auch der zweite Wasserauslass in Fluidverbindung mit dem Wassereinlass (20) steht.

12. Notwaschvorrichtung nach Anspruch 11, wobei der erste voreingestellte Wasserdurchflussratenschwellenwert zwischen 6 Litern pro Minute und 12 Litern pro Minute liegt und der zweite voreingestellte Wasserdurchflussratenschwellenwert einer der Folgenden ist:
zwischen 60 Litern pro Minute und 100 Litern pro Minute; zwischen 30 Litern pro Minute und 60 Litern pro Minute; mindestens 100 Litern pro Minute.

13. Notwaschvorrichtung nach einem der vorhergehenden Ansprüche, wobei die Wascheinheit eine Benutzeridentitätseinheit beinhaltet, die konfiguriert ist, um Identifikationsinformationen zu erzeugen, die einen Benutzer der Wascheinheit identifizieren, und um die Benutzeridentifikationsinformationen zu übertragen;
wobei die Notwaschvorrichtung eine Duschsteuereinheit (300) beinhaltet, die sich lokal an oder entfernt von der Wascheinheit befindet und als Reaktion auf den Empfang der übertragenen Benutzeridentifikationsinformationen ein Telekommunikationssignal ausgibt, das Folgendes übermittelt:
Informationen, die einen Standort der Wascheinheit identifizieren; und Informationen, die den Benutzer identifizieren.

14. Notwaschvorrichtung nach Anspruch 13, wobei die Benutzeridentitätsvorrichtung eines oder mehrere der Folgenden umfasst: einen Fingerabdruckdetektor, der konfiguriert ist, um einen Fingerabdruck des Benutzers der Wascheinheit zu erfassen; eine Kamera, die konfiguriert ist, um das Gesicht des Benutzers der Wascheinheit zu erfassen; einen RFID-Detektor, der konfiguriert ist, um ein RFID-Etikett zu erfassen, das von dem Benutzer der Wascheinheit getragen wird.

15. Notwaschvorrichtung nach Anspruch 13, wobei der Datenprozessor und die Anzeigeeinheit konfiguriert sind, um eines oder jedes der Folgenden zu erzeugen: geografische Koordinaten, die den Standort der Wascheinheit identifizieren; eine Karte einer Umgebung lokal zu der Wascheinheit, auf der der Standort der Wascheinheit angezeigt wird, und um die geografischen Koordinaten oder die Karte auf der grafischen Benutzeroberfläche anzuzeigen.

## Revendications

1. Un appareil de lavage d'urgence apte à générer un aérosol ou un jet contrôlés d'eau directement sur un utilisateur pour qu'il se lave avec, l'appareil de lavage d'urgence comprenant :
une unité de lavage comprenant une entrée d'eau (20) et une ou plusieurs sorties d'eau (2) agencées pour former ledit aérosol ou jet d'eau, reçues en provenance de l'entrée d'eau (20) ;
l'unité de lavage comprenant en outre un réservoir d'eau (100) et un compartiment de lavage (200) au-dessus duquel est disposé le réservoir d'eau, et le réservoir d'eau étant fluidiquement relié à l'entrée d'eau (20) ;
un ou plusieurs capteurs positionnés au niveau de l'entrée d'eau du réservoir d'eau (100) et configurés pour détecter une ou plusieurs propriétés de l'eau reçue au niveau de l'entrée d'eau (20) en provenance du réservoir d'eau (100), les propriétés étant choisis parmi : un débit d'eau, une pression d'eau, une température d'eau, un niveau de pH d'eau, une teneur en bactéries ;
dans lequel les un ou plusieurs capteurs sont configurés pour générer un signal de mesure respectif (22A) indiquant une valeur des une ou plusieurs propriétés détectées de l'eau, et pour transmettre le signal de mesure (22A) pour qu'il soit reçu localement au niveau de, ou à distance de, l'unité de lavage.

2. Un appareil de lavage d'urgence selon la revendication 1, comprenant une unité de contrôle de douche (300) qui est située localement au niveau de, ou à distance de, l'unité de lavage et qui est agencée pour recevoir lesdits signaux de mesure respectifs (22A) et pour délivrer un signal d'alerte (23) si lesdits signaux de mesure (22A) reçus ne vérifient pas des conditions prédéterminées respectives, indiquant ainsi un dysfonctionnement de l'unité de lavage.

3. Un appareil de lavage d'urgence selon la revendication 2, comprenant :
une ou plusieurs soupapes de contrôle de débit d'eau contrôlables à distance, configurées chacune en communication de fluide avec l'entrée d'eau (20) et avec une sortie respective desdites une ou plusieurs sorties d'eau, chaque soupape de contrôle de débit d'eau contrôlable à distance étant actionnable pour placer de façon réversible une sortie respective desdites sorties d'eau en communication de fluide avec l'entrée d'eau (20) ;
dans lequel l'unité de contrôle de douche (300) est agencée pour contrôler à distance lesdites une ou plusieurs soupapes de contrôle de débit d'eau contrôlables à distance pour placer de façon réversible une sortie respective desdites sorties d'eau en communication de fluide avec l'entrée d'eau (20).

4. Un appareil de lavage d'urgence selon la revendication 2 ou la revendication 3, dans lequel l'unité de contrôle de douche (300) est agencée pour délivrer un dit signal d'alerte (23) si une pluralité desdits signaux de mesure (22A) reçus ne vérifient pas en même temps des conditions prédéterminées, de sorte qu'une conformité concomitante avec lesdites conditions prédéterminées soit telle qu'une condition appliquée à l'un desdits signaux de mesure (22A) reçus soit conditionnée par un autre dit signal de surveillance (22A) reçu.

5. Un appareil de lavage d'urgence selon l'une des revendications 2 à 4, dans lequel l'unité de contrôle de douche (300) est située localement au niveau de, ou à distance de, l'unité de lavage et est agencée pour recevoir lesdits signaux de mesure (22A) respectifs, dans lequel l'unité de contrôle de douche (300) comprend un processeur de données et une unité d'affichage configurés pour générer et afficher une interface utilisateur graphique affichant une ou plusieurs desdites propriétés détectées de l'eau.

6. Un appareil de lavage d'urgence selon la revendication 5 ou l'une des revendications précédentes, dans lequel le processeur de données et l'unité d'affichage sont configurés pour délivrer ledit signal d'alerte (23) sous forme d'une alerte affichée par ladite interface utilisateur graphique.

7. Un appareil de lavage d'urgence selon la revendication 3, dans lequel les une ou plusieurs soupapes de contrôle de débit d'eau sont chacune couplées de manière opérante à un commutateur de contrôle respectif agencé pour actionner les une ou plusieurs soupapes de contrôle de débit d'eau pour placer de façon réversible la sortie d'eau en communication de fluide avec l'entrée d'eau (20) ;
un ou plusieurs capteurs sont configurés pour détecter un actionnement de l'un desdits commutateurs de contrôle et pour transmettre un signal de surveillance (22A) indiquant une survenue (30A-C) de l'actionnement ; et
une unité de contrôle de douche (300) est située localement au niveau de, ou à distance de, l'unité de lavage qui est agencée pour recevoir lesdits signaux de surveillance (22A) et lesdits signaux de mesure (22A), et pour délivrer un signal d'alerte (23) si lesdits signaux de mesure (22A) et lesdits signaux de surveillance (22A) reçus ne vérifient pas concomitamment ensemble des conditions prédéterminées, indiquant ainsi un dysfonctionnement de l'unité de lavage.

8. Un appareil de lavage d'urgence selon la revendication 7, dans lequel les un ou plusieurs commutateurs de contrôle sont choisis chacun parmi : des commutateurs de contrôle actionnables à la main.

9. Un appareil de lavage d'urgence selon la revendication 1, comprenant un capteur de débit d'eau agencé pour mesurer un débit d'eau de ladite entrée d'eau (20) vers lesdites une ou plusieurs sorties d'eau, et pour générer un signal de surveillance (22A) indiquant un débit mesuré ;
dans lequel l'unité de contrôle de douche (300) est agencée pour effectuer un recueil de données à distance par :
contrôle d'au moins l'une desdites soupapes de contrôle de débit d'eau contrôlables à distance pour placer l'une respective desdites sorties d'eau en communication de fluide avec l'entrée d'eau (20) pendant une période temporelle prédéterminée finie ; et
réception dudit signal de surveillance (22A) pendant ladite période temporelle.

10. Un appareil de lavage d'urgence selon la revendication 9, dans lequel l'unité de contrôle de douche (300) est agencée pour répéter ledit recueil de données à distance si un intervalle de temps depuis le plus récent recueil de données à distance antérieur dépasse une valeur de seuil de période temporelle.

11. Un appareil de lavage d'urgence selon la revendication 7, comprenant une première desdites sorties d'eau et une seconde desdites sorties d'eau, dans lequel les un ou plusieurs capteurs sont configurés pour détecter ledit débit d'eau et l'unité de contrôle de douche (300) est agencée pour délivrer ledit signal d'alerte (23) si ledit signal de mesure (22A) indique que ledit débit d'eau :
dépasse une première valeur de seuil de débit d'eau prédéterminée et concomitamment l'un desdits signaux de surveillance (22A) indique que seule l'une de la première et de la seconde sortie d'eau est en communication de fluide avec ladite entrée d'eau (20) ; ou
dépasse une seconde valeur de seuil de débit d'eau prédéterminée, qui dépasse la première valeur de seuil de débit d'eau, et concomitamment l'un desdits signaux de surveillance (22A) indique qu'à la fois la première et la seconde sortie d'eau sont en communication de fluide avec ladite entrée d'eau (20).

12. Un appareil de lavage d'urgence selon la revendication 11, dans lequel la première valeur de seuil de débit d'eau prédéterminée est comprise entre 6 litres par minute et 12 litres par minute, et la seconde valeur de seuil de débit d'eau prédéterminée est l'une parmi :
entre 60 litres par minute et 100 litres par minute ; entre 30 litres par minute et 60 litres par minute ; au moins 100 litres par minute.

13. Un appareil de lavage d'urgence selon l'une des revendications précédentes, dans lequel l'unité de lavage comprend une unité d'identité d'utilisateur configurée pour générer une information d'identification identifiant un utilisateur de l'unité de lavage, et pour transmettre l'information d'identification d'utilisateur ;
dans lequel l'appareil de lavage d'urgence comprend une unité de contrôle de douche (300) située localement au niveau de, ou à distance de, l'unité de lavage et qui peut réagir à la réception de l'information d'identification d'utilisateur transmise pour délivrer un signal de télécommunication véhiculant :
une information identifiant un emplacement de l'unité de lavage ; et une information identifiant l'utilisateur.

14. Un appareil de lavage d'urgence selon la revendication 13, dans lequel l'appareil d'identité d'utilisateur comprend l'un quelconque parmi, ou plusieurs parmi : un détecteur d'empreinte digitale configuré pour détecter une empreinte digitale de l'utilisateur de l'unité de lavage ; une caméra configurée pour détecter le visage de l'utilisateur de l'unité de lavage ; un détecteur RFID configuré pour détecter une étiquette RFID portée par l'utilisateur de l'unité de lavage.

15. Un appareil de lavage d'urgence selon la revendication 13, dans lequel le processeur de données et l'unité d'affichage sont configurés pour générer l'une d'entre, ou chacune parmi : des coordonnées géographiques identifiant l'emplacement de l'unité de lavage ; une carte d'un environnement local de l'unité de lavage sur laquelle est présenté l'emplacement de l'unité de lavage, et pour afficher les coordonnées géographiques ou la carte sur ladite interface utilisateur graphique.
